# EUROPÄISCHE PATENTSCHRIFT

(11) **EP 1 034 173 B1**
(45) Veröffentlichungstag und Bekanntmachung des Hinweises auf die Patenterteilung: **27.04.2005**
(21) Anmeldenummer: 98959874.3
(22) Anmeldetag: 12.11.1998
(51) Int. Cl.: C07D 401/00

(54) **VERFAHREN ZUR HERSTELLUNG VON 8-METHOXY-CHINOLONCARBONSÄUREN**
METHOD FOR PRODUCING 8-METHOXY-QUINOLINE CARBOXYLIC ACIDS
PROCEDE DE PRODUCTION D'ACIDES CARBOXYLIQUES DE 8-METHOXY-QUINOLONE

(30) Priorität: 24.11.1997 DE 19751948
(43) Veröffentlichungstag der Anmeldung: 13.09.2000
(73) Patentinhaber: Bayer HealthCare AG, 51368 Leverkusen (DE)
(72) Erfinder: GEHRING, Reinhold, D-42327 Wuppertal (DE); MOHRS, Klaus, D-42113 Wuppertal (DE); HEILMANN, Werner, D-42327 Wuppertal (DE); DIEHL, Herbert, D-51373 Leverkusen (DE)
(86) Internationale Anmeldenummer: PCT/EP1998/007237
(87) Internationale Veröffentlichungsnummer: WO 1999/026940

(56) Entgegenhaltungen:
- EP-A- 0 106 489
- EP-A- 0 230 295
- EP-A- 0 235 762
- EP-A- 0 342 675
- EP-A- 0 550 903
- EP-A- 0 591 808
- DE-A- 4 234 330
- DE-A- 19 546 249

## Beschreibung

Die Erfindung betrifft ein Verfahren zur Herstellung von 8-Methoxy-Chinoloncarbonsäuren.

8-Methoxy-Chinoloncarbonsäuren stellen Antibiotika mit hoher antibakterieller Wirkung gegen gram-negative und gram-positive Bakterien dar. So weisen beispielsweise die Antibiotika 1-Cyclopropyl-6-fluor-1,4-dihydro-8-methoxy-7-(3-methyl-1-piperazinyl)-4-oxo-3-chinolincarbonsäure (INN, Gatifloxacin, EP-A-230 295) und 1-Cyclopropyl-7-[S,S]-2,8-diazabicyclo[4.3.0]non-8-yl)-6-fluor-1,4-dihydro-8-methoxy-4-oxo-3-chinoloncarbonsäure-hydrochlorid-monohydrat (Bay 12-8039, EP-A-0 350 733) in der 8-Stellung eine Methoxygruppe auf.

Solche antibakteriell hochwirksamen Chinoloncarbonsäuren weisen in der Regel einen heteromonocyclischen oder heteropolycyclischen Amin-Rest in der 7-Position der Chinoloncarbonsäure auf Dieser cyclische Amin-Rest wird im allgemeinen durch nucleophile Substitution der entsprechenden 7-Halogen-Chinoloncarbonsäure mit dem jeweiligen Amin hergestellt. Die Einführung der 8-Alkoxygruppe kann prinzipiell vor Einführung des cyclischen Amin-Restes in der 7-Position oder danach erfolgen. So beschreibt die EP-A-0 350 733 die Herstellung des racemischen Betains des oben erwähnten Bay 12-8039, ausgehend von der entsprechenden 8-Methoxyverbindung, deren Herstellung in der EP-A-0 241 206 (Präparat 6) beschrieben ist, durch nucleophile Substitution mit dem entsprechenden racemischen Amin. Analog ist die Herstellung des enantiomerenreinen Betains des Bay 12-8039, ausgehend von der 8-Methoxyverbindung durch nucleophile Substitution mit dem enantiomerenreinen Amin in der EP-A-0 550 903 (Beispiel 19) beschrieben. Der dort beschriebene Herstellungsweg erfordert jedoch eine aufwendige Isolierung und Reinigung durch Säulenchromatographie, die für den technischen Maßstab unerwünscht ist. Letzterer Weg wird auch in der EP-A-0 591 808 (Beispiel Z 19) beschritten.

Ein anderer Weg der Einführung eines 8-Alkoxysubstituenten in die 7-aminsubstituierten Chinoloncarbonsäuren besteht in der 8-Alkoxysubstitution, nachdem man den cyclischen Amin-Substituenten in die 7-Position aus der entsprechenden 7,8-Dihalogen-Ausgangsverbindung erhalten hat.

So beschreibt die EP-A-0 106 489 den Weg der 8-Methoxysubstitution nach Einführung des Heterocyclyl-Substituenten in 7-Position durch Umsetzung der entsprechenden 8-Fluorverbindung in Methanol in Gegenwart von Kalium-tert.-butoxid. Die dort mit der 7-[2-[(Methylamino)-methyl]-4-thiazol]-Verbindung durchgeführte Reaktion erfordert jedoch 24 Stunden unter Rückfluß und ist damit für eine Umsetzung im technischen Maßstab ungeeignet. Außerdem zeigt sich, daß bestimmte Chinoloncarbonsäuren, wie beispielsweise das oben beschriebene Bay 12-8039 auf diesem Weg nicht hergestellt werden können, da unter den Bedingungen der Umsetzung unter Rückfluß für 24 Stunden keine Reaktion stattfindet.

Den Weg der 8-Alkoxy-Substitution ausgehend von 8-Halogen-7-monocycloamin-Derivaten in Methanol in Gegenwart von Alkalimetallalkoholaten beschreibt auch die EP-A-02 30 295. Die dort in den Beispielen beschriebene Umsetzung in Gegenwart von Natriummethanolat erfordert jedoch sehr hohe Temperaturen von ca. 140 bis 150°C und sehr lange Reaktionszeiten, und die Reaktion wird in verschlossenen Gefäßen unter Druck durchgeführt. Dieses Verfahren ist jedoch nicht allgemein zur Herstellung von 8-Methoxy-Chinoloncarbonsäuren anwendbar. So führt die Anwendung dieses Verfahrens auf die Herstellung des oben beschriebenen Bay 12-8039 selbst nach 70 Stunden nicht zur Bildung des Endproduktes, wenn MeOH als Lösungsmittel eingesetzt wird.

Wird die Umsetzung der entsprechenden 8-Fluorverbindung mit Natriummethanolat in Tetrahydrofuran durchgeführt, sind für eine vollständige Umsetzung sehr lange Reaktionszeiten (>24 h) und ein großer Methanolat-Überschuß notwendig.

In ähnlicher Weise erfolgt die Herstellung der 8-Alkoxyderivate in der EP-A-0 235 762 durch Umsetzung der 8-Halogen-7-monocycloamin-Derivate mit Alkalimetallalkoholaten. Desweiteren ist die Herstellung von 8-Methoxy-Chinoloncarbonsäuren durch Umsetzung der Alkalimetallalkoholate in Lösungsmitteln wie DMI (WO 93/22308, Chugai), mit Natriummethanolat in DMF oder DMSO (EP-A-0 342 675, Chugai), mit Benzylalkohol/Natriumhydrid (Research Disclosure No. 291 097, 1988), mit Natriummethanolat in DMF bei 80°C für 9 Stunden (JP 03007283 Yoshitomi), mit Methanol und einer Base (WO 90/06305, Dainippon), mit NaH/Trifluorethanol in DMF (WO 92/09579), mit Natriummethanolat in Methanol (JP-62 252772), mit Natriummethanolat/DMI bei 80°C (Jp-05117 238, Chugai) sowie die Umsetzung mit Natriummethanolat in Methanol (J. Med. Chem. 30, 2163-2169) beschrieben.

Zwar führt die Umsetzung der 8-Halogenverbindungen mit Alkalimetallalkoholaten in polaren, aprotischen Lösungsmitteln wie z.B. DMF im allgemeinen zu einer praktisch vollständigen Umsetzung, falls das Alkalimetallalkoholat im Überschuß verwendet wird, die Isolierung von Salzen der 8-Alkoxy-Chinoloncarbonsäuren ist jedoch aus solchen Lösungsmitteln aufwendig und technisch kaum zu realisieren.

Die Aufgabenstellung der vorliegenden Erfindung besteht somit darin, ein Verfahren zur Herstellung von 8-Methoxyderivaten von Chinoloncarbonsäuren zu entwickeln, das kurze Reaktionszeiten, das Arbeiten unter Atmosphärendruck eine vollständige Umsetzung sowie die leichte Aufarbeitung des Reaktionsgemischs ermöglicht, zu entwickeln.

Überraschenderweise gelingt es, 8-Methoxy-Chinoloncarbonsäure-Derivate in einem Verfahren, das den obigen Anforderungen genügt, durch Umsetzung der entsprechenden 8-Halogen-Chinoloncarbonsäure-Derivate mit (C₁-C₃)-Alkanolen oder Benzylalkohol und Natrium- oder Kalium-tert.-butylat oder Natrium- oder Kalium-tert.amylat in Gegenwart von aliphatischen oder cycloaliphatischen Ethern mit 4 bis 6 Kohlenstoffatomen als Lösungsmittel, zu erhalten.

Gegenstand der Erfindung ist somit ein Verfahren zur Herstellung von Verbindungen der Formel 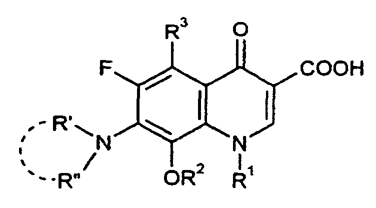 in der
- R' und R": zusammen mit dem Stickstoffatom, an das sie gebunden sind einen mono- oder bicyclischen Heterocyclus bilden, der gegebenenfalls in allen Ringteilen weitere Stickstoff-, Sauerstoff- oder Schwefel-Heteroatome enthalten kann und der gegebenenfalls substituiert sein kann,
in welcher
- R¹: für C₁-C₃-Alkyl, FCH₂-CH₂-, Cyclopropyl, gegebenenfalls durch Halogen ein- bis dreifach substituiertes Phenyl oder Cyclopropyl,
- R²: für C₁-C₃-Alkyl oder Benzyl,
- R³: für H, Halogen, NH₂, CH₃ steht,
dadurch gekennzeichnet, daß man 8-Halogen-3-chinoloncarbonsäurederivate der allgemeinen Formel 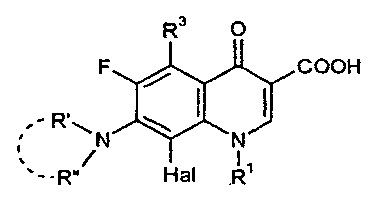 in welcher
- Hal: für Fluor oder Chlor steht und
R¹, R², R³ und 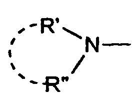 die oben angegebene Bedeutung haben,
in einem aliphatischen oder cycloaliphatischen Ether mit 4 bis 6 Kohlenstoffatomen als Lösungsmittel in Gegenwart von C₁-C₃-Alkanolen oder Benzylalkohol mit 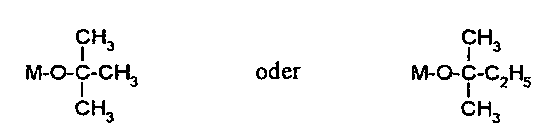 worin
- M: für Natrium oder Kalium steht,
umsetzt.

Die Gruppe 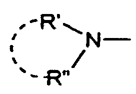 bildet einen mono- oder bicyclischen Heterocyclus, der gegebenenfalls in allen Ringteilen weitere Stickstoff-, Sauerstoff- oder Schwefel-Heteroatome enthalten kann und gegebenenfalls substituiert sein kann. Die Ringglieder R' und R" können darin gleiche oder verschiedene Ringbestandteile darstellen. Solche mono- oder bicyclischen Aminreste in der 7-Position des Chinoloncarbonsäuregrundkörpers sind auf dem Gebiet der Chinoloncarbonsäureantibiotika grundsätzlich bekannt. Beispielhaft sei hier verwiesen auf die Patentveröffentlichungen EP-A-0 523 512, EP-A-0 230 295, EP-A-0 705 828, EP-A-0 589 318, EP-A-0 357 047, EP-A-0 588 166, GB-A-2 289 674, WO 92/09 579, JP-03-007 283, EP-A-0 241 206, EP-A-0 342 675, WO 93/22 308 und EP-A-0 350 733.

Unter diesen bekannten Aminresten steht 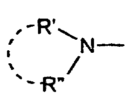 bevorzugt für

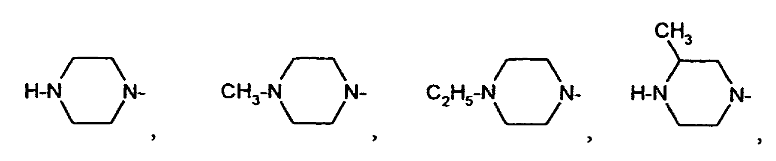

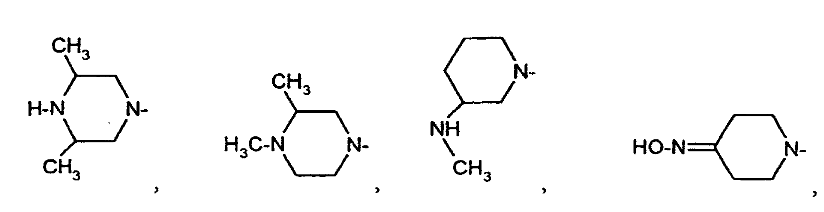

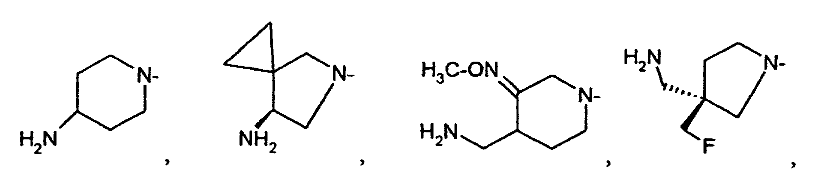

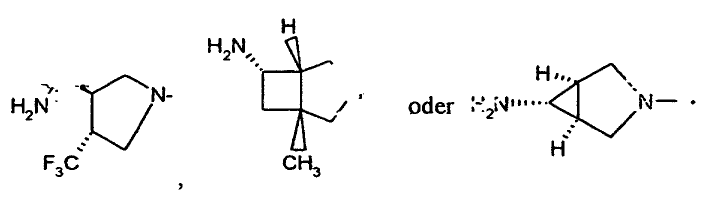

Ganz besonders bevorzugt steht 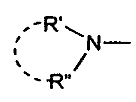 für 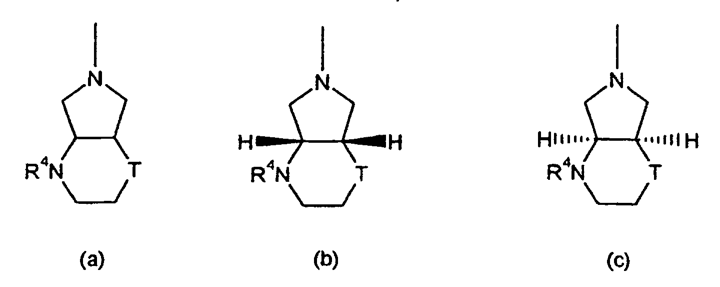 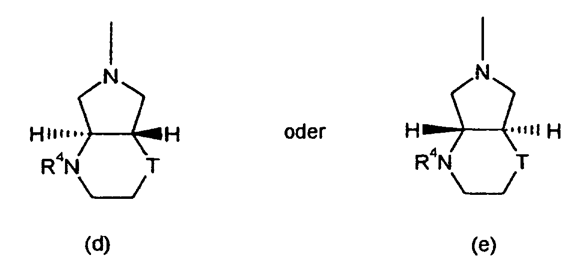 in welchen
- T: für -O- oder -CH₂- und
- R⁴: für Wasserstoff, C₁-C₃-Alkyl, C₂-C₅-Oxoalkyl, -CH₂-CO-C₆H₅, -CH₂CH₂CO₂R⁵, 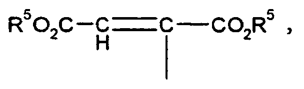 5-Methyl-2-oxo-1,3-dioxol-4 yl-methyl, -CH=CH-CO₂R⁵ oder -CH₂CH₂-CN,
steht, worin
R⁵ Wasserstoff oder C₁-C₃-Alkyl bedeutet,
und die Formel (a) beliebige Mischungen der Stereoisomere (b) bis (e) einschließt.

Die diesen Definitionen von 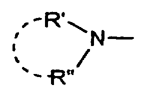 entsprechenden Amine sind in der EP-A-0 550 903 beschrieben, und ihre Umsetzung mit den entsprechenden 6,7,8-Trihalogen-Chinoloncarbonsäuren führt zu den Ausgangsverbindungen des erfindungsgemäßen Verfahrens.

Der aliphatische oder cycloaliphatische Ether mit 4 bis 6 Kohlenstoffatomen wird bevorzugt aus Dimethoxyethan, Dioxan und Tetrahydrofuran ausgewählt.

Besonders hohe Ausbeuten und kurze Reaktionszeiten werden mit Tetrahydrofuran erzielt.

Bevorzugt steht Hal in den erfindungsgemäßen Verfahren für Fluor.

Bevorzugt ist der (C₁-C₃)-Alkanol Methanol, d.h. das Verfahren wird bevorzugt zur Herstellung der 8-Methoxyverbindung angewendet.

M ist bevorzugt Kalium, d.h. die Umsetzung erfolgt bevorzugt mit Kalium-tert.butylat oder -amylat, besonders bevorzugt für Kalium-tert.-butylat.

Bezogen auf 1 Äquivalent der Verbindung der Formel 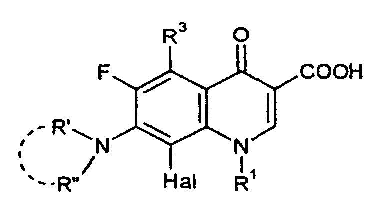 werden bevorzugt 1 bis 3, besonders bevorzugt 1,1 bis 1,3 Äquivalente des (C₁-C₃)-Alkanols oder des Benzylalkohols, und 2 bis 3, bevorzugt 2,1 bis 2,3 Äquivalente der Verbindung der Formel 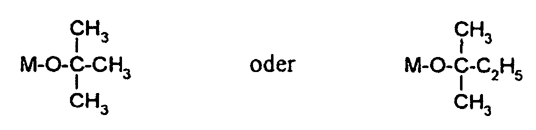 eingesetzt.

Die Reaktion wird bevorzugt zwischen 20°C und der Siedetemperatur des Lösungsmittels bei Normaldruck durchgeführt.

Das Verfahren der vorliegenden Erfindung eignet sich besonders zur Herstellung von Verbindungen der Formel 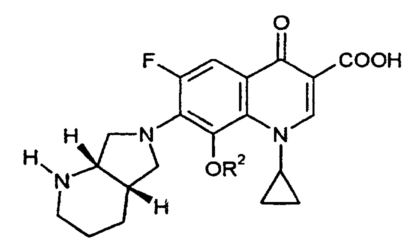 worin
- R²: für C₁-C₃-Alkyl oder Benzyl steht.

Bevorzugt setzt man dabei 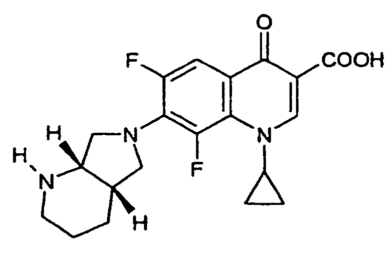 in einem aliphatischen oder cycloaliphatischen Ether mit 4 bis 6 Kohlenstoffatomen als Lösungsmittel in Gegenwart von C₁-C₃-Alkoholen oder Benzylalkohol mit 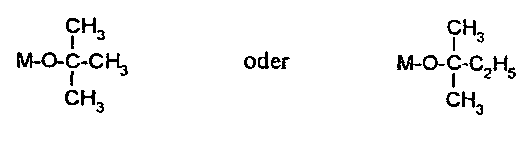 worin
- M: für Natrium oder Kalium steht,
um.

Das Verfahren wird bevorzugt in Dimethoxyethan, Dioxan, Tetrahydrofuran oder Mischungen daraus durchgeführt.

Besonders bevorzugt wird das Verfahren in Tetrahydrofuran als Lösungsmittel durchgeführt.

Bevorzugt ist der (C₁-C₃)-Alkanol Methanol, d.h. die 8-Methoxyverbindung wird hergestellt (Bay 12-8039).

M ist bevorzugt Kalium.

Bevorzugt werden, bezogen auf 1 Äquivalent der Verbindung der Formel 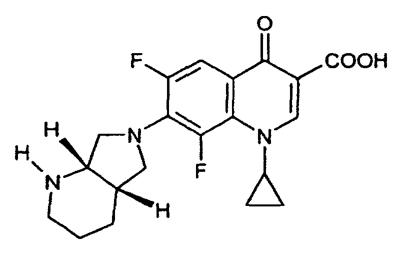 1 bis 3, besonders bevorzugt 1,1 bis 1,3 Äquivalente des (C₁-C₃)-Alkanols oder des Benzylalkohols, und 2 bis 3, bevorzugt 2,1 bis 2,3 Äquivalente der Verbindung der Formel 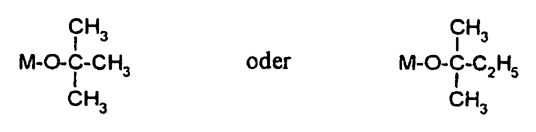 eingesetzt.

Das Verfahren wird bevorzugt zwischen 20°C und der Siedetemperatur des Lösungsmittels bei Normaldruck durchgeführt.

Das Verfahren der vorliegenden Erfindung eignet sich besonders für die Herstellung von

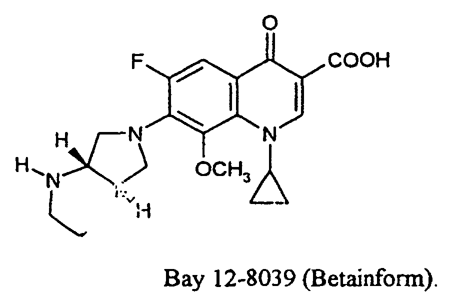

Dabei setzt man 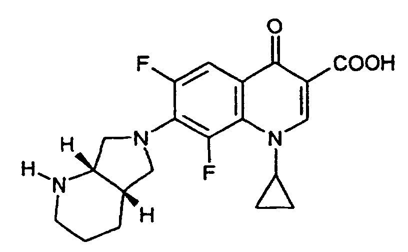 mit Methanol und bevorzugt Kalium-tert.-butylat in Tetrahydrofuran als Lösungsmittel um.

Bezogen auf ein Äquivalent der Verbindung der Formel 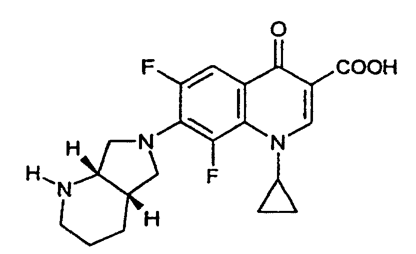 werden bevorzugt 1 bis 3, besonders bevorzugt 1,1 bis 1,3 Äquivalente Methanol und 2 bis 3, bevorzugt 2,1 bis 2,3 Äquivalente Kalium-tert.-butylat eingesetzt und die Reaktion wird zwischen 20°C und der Siedetemperatur des Lösungsmittels bei Normaldruck durchgeführt.

Ein besonderer Vorzug des erfindungsgemäßen Verfahrens besteht darin, daß die Herstellung pharmazeutisch verträglicher Salze der oben beschriebenen Verbindungen, beispielsweise der Hydrochloride, besonders leicht durch Versetzen der erhaltenen Reaktionsmischung mit verdünnter Salzsäure oder Zugabe der Reaktionsmischung zu verdünnter Salzsäure und Isolation des Salzes, bevorzugt des Hydrochlorids, durch Filtration, gelingt. Diese sofortige Herstellung des Hydrochlorids wird bevorzugt zur Herstellung der Verbindung der folgenden Formel angewendet:

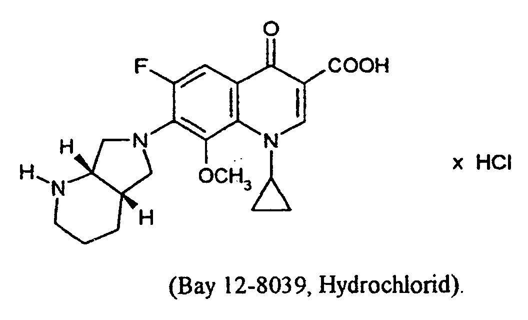

In einem weiteren Aspekt der vorliegenden Erfindung kann die zuvor beschriebene Verbindung (Bay 12-8039, Hydrochlorid) überraschend in hoher Reinheit durch Umkristallisation aus Wasser oder einem Wasser/(C₁-C₃)-Alkanolgemisch isoliert werden. Die Reinheit der so erhaltenen Verbindung ist für viele pharmazeutische Anwendungen bereits ausreichend. Bevorzugt erfolgt die Umkristallisation aus Wasser oder einem Wasser/Ethanol-Gemisch.

Aus dem zuvor beschriebenen Hydrochlorid kann weiterhin überraschend in einfacher Weise im technischen Maßstab ein besonders stabiles Mono-Hydrat der Formel 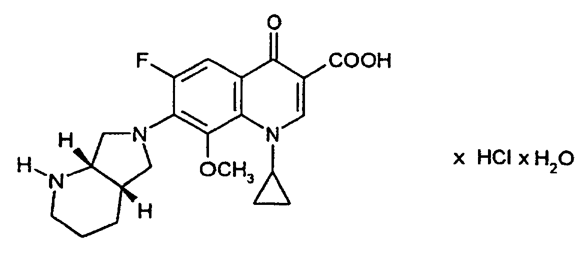 mit einer bestimmten Kristallstruktur, wie sie in der DE-A-1 95 46 249 (entsprechend EP-A-0 780 390) beschrieben ist, durch Trocknung des erhaltenen Produktes bei 40 bis 60°C und 80 bis 120 mbar erhalten werden. Besonders bevorzugt wird diese Trocknung bei etwa 50°C und etwa 100 mbar durchgeführt.

(C₁-C₃)-Alkyl bzw. -Alkylreste in den obigen Definitionen steht im allgemeinen für beispielsweise Methyl, Ethyl, Propyl, Isopropyl.

Besonders bevorzugt stellt (C₁-C₃)-Alkyl und der (C₁-C₃)-Alkylrest in den entsprechenden aliphatischen Resten Methyl dar.

### Beispiel 1

Herstellung von 1-Cyclopropyl-7-([S,S]-2,8-diazabicyclo[4.3.0]non-8-yl)-6-fluor-1,4-dihydro-8-methoxy-4-oxo-3-chinoloncarbonsäure-hydrochlorid unter Verwendung von Kalium-tert.-butylat

### Einsatzmengen:

| | | |
|---|---|---|
| 50,0 g | (0,129 mol) | 1-Cyclopropyl-7-([S,S]-2,8-diazabicyclo[4.3.0]non-8- |
| | | yl)-6,8-difluor-1,4-dihydroxy-4-oxo-3-chinolocarbonsäure (Bay z 7906) (hergestellt gemäß Beispiel 1 der EP-A-0 550 903). |
| 270,0 ml | | THF |
| 6,2 ml | (0, 155 mol) | Methanol |
| 159,1 g | (0,284 mol) | Kalium-tert.-butylat-Lösung (20 %ig in THF) |
| 128 ml | | Wasser |
| 38 ml | | Salzsäure, konz. |
| 30 ml | | Wasser |
| 60 ml | | Ethanol |
| 110 ml | | Ethanol |
| 330 ml | | Wasser |
| 3 x 15 ml | | Ethanol |

### Arbeitsweise:

In einem 1000 ml Dreihalskolben mit Rührer und Thermometer wurden unter Stickstoff 50,0 g Bay z 7906 in 270 ml THF und 6,2 ml Methanol vorgelegt. Es wird erwärmt und ab ca. 60°C in ungefähr 5 min 80 ml Kalium-tert.-butytat-Lösung (20 %ig in THF), entsprechend 1 Äquivalent, zulaufen gelassen. Es entsteht eine gelbe Suspension, die langsam dickflüssiger wird und schließlich weiß ist. Es wird 15 min unter Rückfluß gerührt. Dabei zeigt die Suspension keine Veränderung. Unter Rückfluß wird innerhalb 5 min die restliche Kalium-tert.-butylat-Lösung zulaufen gelassen. Es wird 2,5 Stunden bei Rückfluß gerührt und anschließend auf Raumtemperatur abgekühlt.

Zur Fällung des Hydrochlorids werden 128 ml Wasser und 38 ml konz. Salzsäure in einem Doppelmantelkolben mit Rührer, Drehzahlmesser und Thermostat vorgelegt. Bei 500 U/min wird unter Kühlen innerhalb von 2 Stunden bei ca. 20 bis 22°C die oben erhaltene Reaktionslösung zugetropft. Nach der Zugabe von ca. 50 ml wird mit 12,5 mg Bay 12-8039 geimpft. Nach der vollständigen Zugabe wird ca. 30 min bei 8°C gerührt. Es bildet sich eine Suspension. Es wird filtriert und erst mit 30 ml Wasser und dann mit 60 ml Ethanol gewaschen. Das ausgefallene Endprodukt läßt sich sehr gut filtrieren und wird im Vakuum bei 50°C getrocknet.

Es werden 47,1 g des Produktes erhalten.

### Reinigung und Monohydratbildung

Zur Reinigung werden 46,6 g des gefällten Produktes in 110 ml Ethanol / 330 ml Wasser unter Rückfluß gelöst, und man läßt innerhalb von 2 Stunden auf 20 bis 22°C abkühlen. Bei etwa 50°C wird mit 12,5 mg Bay 12-8039 angeimpft. Die Impfkristalle gehen nicht in Lösung. Es wird noch eine weitere Stunde bei 20 bis 22°C gerührt und filtriert und mit 3 x 15 ml Ethanol gewaschen. Die Trocknung bei etwa 50°C und 100 mbar Druck führt zur definierten Bildung des Monohydrats von Bay 12-8039-Hydrochlorid.

Es werden 31,9 g eines gelben Kristallisates erhalten, daß für viele pharmakologische Anwendungen eine ausreichende Reinheit besitzt.

### Beispiel 2

Herstellung von 1-Cyclopropyl-7-([S,S]-2,8-diazabicyclo[4.3.0]non-8-yl)-6-fluor-1,4-dihydro-8-methoxy-4-oxo-3-chinoloncarbonsäure-hydrochlorid unter Verwendung von Kalium-tert.-amylat

### Einsatzmengen:

| | | |
|---|---|---|
| 10,0 g | (25,7 mmol) | Bay z 7906, Pt. 501781 |
| 54 ml | | THF |
| 1,24 ml | (30,8 mmol) | Methanol |
| 35,6 g | (56,5 mmol) | Kalium-tert.-amylat-Lösung (20 %ig in THF) |
| 26,6 ml | | Wasser |
| 7,6 ml | | Salzsäure, konz. |
| 6,0 ml | | Wasser |
| 12,0 ml | | Ethanol |

### Arbeitsweise:

10 g Bay z 7906 werden in 54 ml THF und 1,24 ml Methanol vorgelegt. Es wird aufgeheizt und ab 60°C in ca. 5 Minuten 17 ml Kalium-tert.-amylat-Lösung (20 %ig in THF) entsprechend 1 Äquivalent zulaufen gelassen. Dann wird 30 Minuten unter Rückfluß gerührt. Unter Rückfluß wird in 5 Minuten die restliche Kalium-tert.amylat-Lösung zulaufen gelassen. Es wird 2,5 Stunden bei Rückfluß gerührt und anschließend auf Raumtemperatur abgekühlt. Zur Fällung des Hydrochlorids werden 26,6 ml Wasser und 7,6 ml Salzsäure (konz.) vorgelegt. Bei 500 U/Min. wird unter Kühlen in 2 Stunden bei ca. 20 bis 22°C die Reaktionslösung zugetropft. Nach Zugabe von ca. 9 ml wird mit Bay 128039 angeimpft. Nach der vollständigen Zugabe wird 30 Minuten bei 8°C gerührt. Es bildet sich eine Suspension. Es wird filtriert und erst mit 6 ml Wasser und dann mit 12 ml Ethanol gewaschen und bei 50°C im Vakuum getrocknet. Es werden 8,6 g erhalten.

Das Produkt kann analog zu Beispiel 1 gereinigt werden und in das Monohydrat überführt werden.

### Vergleichsbeispiel:

### (Umsetzung von Bay z 7906 in THF mit Natriummethylat)

### Einsatzmengen:

| | | |
|---|---|---|
| 50,0 g | (0,129 mol) | Bay z 7906 |
| 1040 ml | | THF |
| 116,1 g | (0,645 mol) | Natriummethylat-Lösung (30 %ig in Methanol) |
| 49 ml | | Salzsäure, konz. |
| 77 ml | | THF |
| 38 ml | | Wasser |
| 385 ml | | Wasser |
| 2 x 38 ml | | Wasser |
| 297 ml | | Methanol |
| 2 x 10 ml | | Methanol |
| 68 ml | | Ethanol |
| 34 ml | | Wasser |
| 2 x 10 ml | | Ethanol |

### Arbeitsweise:

In einem 2000 ml Dreihalskolben mit Rührer und Thermometer werden unter Stickstoff 50,0 g Bay z 7906 in 1040 ml THF und 116,1 g Natriummethylat-Lösung (30 %ig in Methanol) vorgelegt. Das Gemisch wird unter Rühren zum Rückfluß erhitzt und der Reaktionsverlauf mittels HPLC-Kontrolle verfolgt.

(Bay z 7906 [Ausgangsmaterial]-Gehalt:
nach 6 h RF = 56,5 %;
nach 30 h RF = 11,7 %;
nach 70 h RF = 1,4 %).

Nach 70 Stunden Rühren unter Rückfluß wird auf 10 bis 15°C abgekühlt und mit konz. Salzsäure auf pH 6,8 bis 7,0 gestellt (Verbrauch konz. Salzsäure 48 ml). Der Niederschlag wird abgesaugt und zuerst mit 77 ml THF, dann mit 38 ml Wasser gewaschen.

Der feuchte Feststoff (108,2 g) wird in 385 ml Wasser suspendiert, 30 Minuten bei 20 bis 25°C gerührt, abgesaugt und zweimal mit 38 ml Wasser gewaschen (schlechtes Saugverhalten).

Der bei 50°C im Vakuum getrocknete Feststoff (40,4 g) wird in 297 ml Methanol bei Rückfluß gelöst.

Nach Abkühlen auf ca. 10°C werden die ausgefallenen Kristalle abgesaugt und zweimal mit je 10 ml Methanol gewaschen.

Der bei 50°C im Vakuum getrocknete Feststoff (21,1 g) wird in 68 ml Ethanol und 34 ml Wasser bei Rückfluß gelöst. Nach Abkühlen auf 20 bis 25°C wird eine Stunde nachgerührt, die ausgefallenen Kristalle abgesaugt und zweimal mit 10 ml Ethanol nachgewaschen. Nach Trocknen bei 50°C im Vakuum werden 16,2 g oranges Kristallisat erhalten.

Der Vergleich zwischen den erfindungsgemäß Beispielen und dem Vergleichsbeispiel zeigt, daß selbst nach 70 h Reaktionszeit bei der Umsetzung mit Natriummethylat in THF eine geringere Ausbeute erhalten wird.

Das erfindungsgemäße Verfahren bietet somit insbesondere im industriellen Maßstab enorme Vorteile bei Ausbeute, Reaktionsdauer und Aufarbeitung.

## Patentansprüche

1. Verfahren zur Herstellung von 3-Chinoloncarbonsäurederivaten der allgemeinen Formel 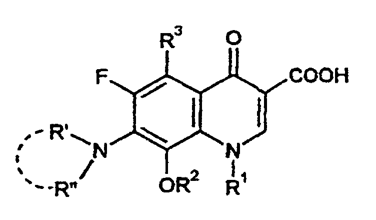 in der
R' und R" zusammen mit dem Stickstoffatom, an das sie gebunden sind, einen mono- oder bicyclischen Heterocyclus bilden, der gegebenenfalls in allen Ringteilen weitere Stickstoff-, Sauerstoff- oder Schwefel-Heteroatome enthalten kann und der gegebenenfalls substituiert sein kann,
in welcher
R¹ für C₁-C₃-Alkyl, FCH₂-CH₂-, Cyclopropyl, gegebenenfalls durch Halogen ein- bis dreifach substituiertes Phenyl oder Cyclopropyl,
R² für C₁-C₃-Alkyl oder Benzyl,
R³ für H, Halogen, NH₂, CH₃ steht,
**dadurch gekennzeichnet, daß** man 8-Halogen-3-chinoloncarbonsäurederivate der allgemeinen Formel 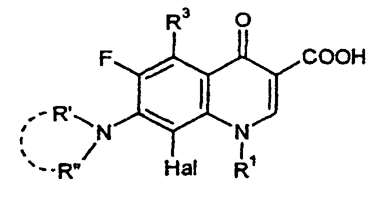 in welcher
Hal für Fluor oder Chlor steht und R¹, R³ und 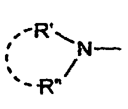 die oben angegebene Bedeutung haben,
in einem aliphatischen oder cycloaliphatischen Ether mit 4 bis 6 Kohlenstoffatomen als Lösungsmittel in Gegenwart von C₁-C₃-Alkanolen oder Benzylalkohol mit 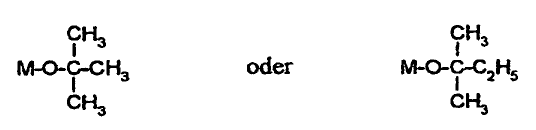 worin
M für Natrium oder Kalium steht,
umsetzt.

2. Verfahren nach Anspruch 1, **dadurch gekennzeichnet, daß** das Lösungsmittel aus der Gruppe ausgewählt wird, die aus Dimethoxyethan, Dioxan und Tetrahydrofuran besteht.

3. Verfahren nach Anspruch 2, **dadurch gekennzeichnet, daß** das Lösungsmittel Tetrahydrofuran ist.

4. Verfahren nach irgendeinem der Ansprüche 1 bis 3, **dadurch gekennzeichnet, daß** Hal für Fluor steht.

5. Verfahren nach irgendeinem der Ansprüche 1 bis 4, **dadurch gekennzeichnet, daß** das (C₁-C₃)-Alkanol Methanol ist.

6. Verfahren nach irgendeinem der Ansprüche 1 bis 5, **dadurch gekennzeichnet, daß** M Kalium ist.

7. Verfahren nach irgendeinem der Ansprüche 1 bis 6, **dadurch gekennzeichnet, daß** bezogen auf 1 Äquivalent der Verbindung der Formel 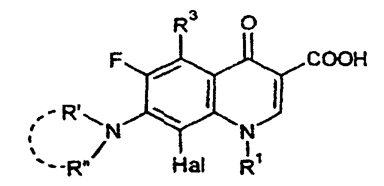 1 bis 3 Äquivalente des (C₁-C₃)-Alkanols oder des Benzylalkohols und 2 bis 3 Äquivalente der Verbindung der Formel 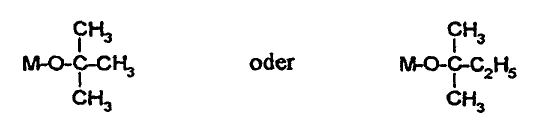 eingesetzt werden.

8. Verfahren nach irgendeinem der Ansprüche 1 bis 7, **dadurch gekennzeichnet, daß** man die Reaktion zwischen 20°C und der Siedetemperatur des Lösungsmittels bei Normaldruck durchführt.

9. Verfahren zur Herstellung von 3-Chinoloncarbonsäurederivaten nach irgendeinem der Ansprüche 1 bis 8, worin. 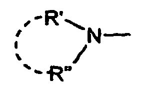 für 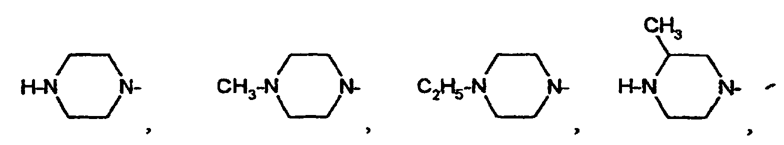 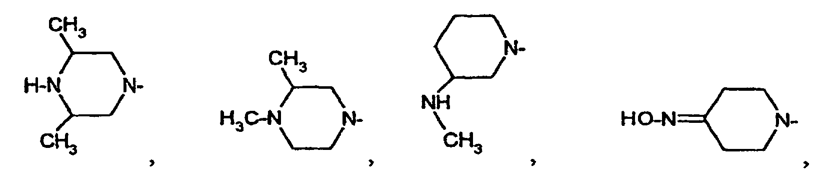 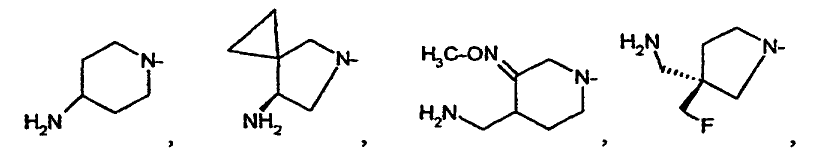 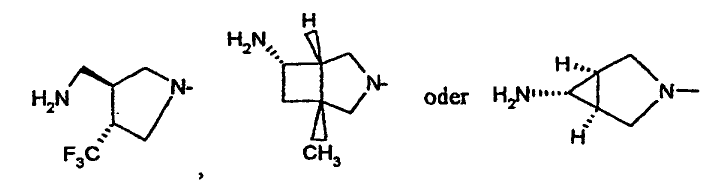 steht.

10. Verfahren zur Herstellung von 3-Chinoloacarbonsäurederivaten nach irgendeinem der Ansprüche 1 bis 8, worin 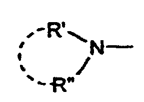 für 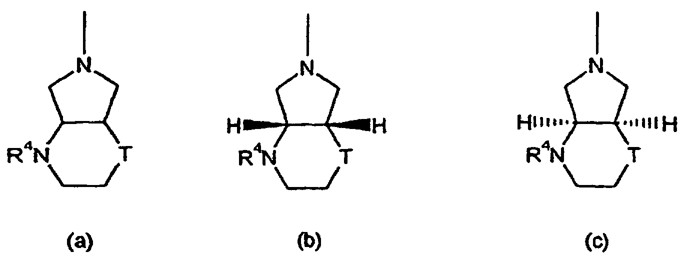 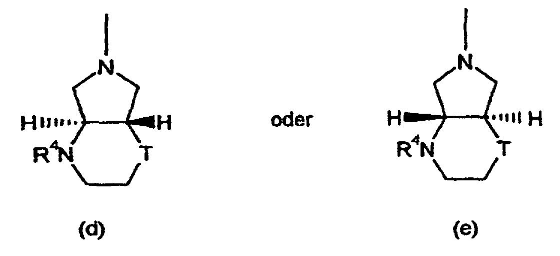 steht, in welchen
T für -O- oder -CH₂- und
R⁴ für Wasserstoff, C₁-C₃-Alkyl, C₂-C₅-Oxoalkyl, -CH₂-CO-C₆H₅, -CH₂CH₂CO₂R⁵, 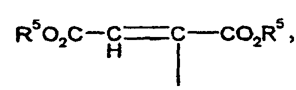 5-Methyl-2-oxo-1,3-dioxol-4-yl-methyl, -CH=CH-CO₂R⁵ oder -CH₂CH₂-CN,
steht, worin
R⁵ Wasserstoff oder C₁-C₃-Alkyl bedeutet,
und die Formel (a) beliebige Mischungen der Stereoisomere (b) bis (e) einschließt.

11. Verfahren zur Herstellung von 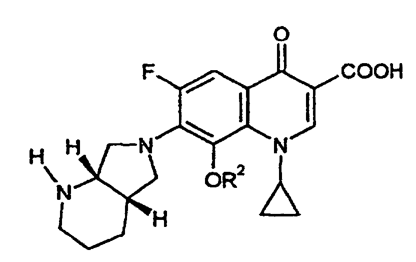 worin
R² für C₁-C₃-Alkyl oder Benzyl steht,
**dadurch gekennzeichnet, daß** man 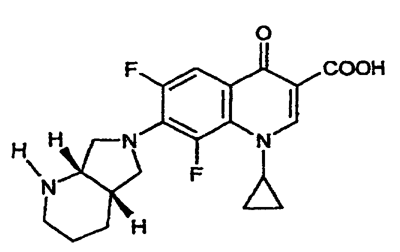 in einem aliphatischen oder cycloaliphatischen Ether mit 4 bis 6 Kohlenstoffatomen als Lösungsmittel in Gegenwart von C₁-C₃-Alkoholen oder Benzylalkohol mit 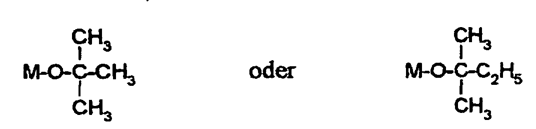 worin
M für Natrium oder Kalium steht,
umsetzt.

12. Verfahren nach Anspruch 1, **dadurch gekennzeichnet, daß** das Lösungsmittel aus der Gruppe ausgewählt wird, die aus Dimethoxyethan, Dioxan und Tetrahydrofuran besteht.

13. Verfahren nach Anspruch 11, **dadurch gekennzeichnet, daß** das Lösungsmittel Tetrahydrofuran ist.

14. Verfahren nach Anspruch 11, **dadurch gekennzeichnet, daß** das (C₁-C₃)-Alkanol Methanol ist.

15. Verfahren nach Anspruch 11, **dadurch gekennzeichnet, daß** M Kalium ist.

16. Verfahren nach Anspruch 11, **dadurch gekennzeichnet, daß** bezogen auf 1 Äquivalent der Verbindung der Formel 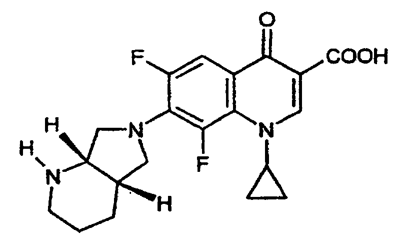 1 bis 3 Äquivalente des (C₁-C₃)-Alkanols oder des Benaylalkohols und 2 bis 3 Äquivalente der Verbindung der Formel 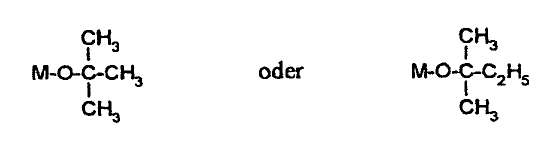 eingesetzt werden.

17. Verfahren nach Anspruch 11, **dadurch gekennzeichnet, daß** man die Reaktion zwischen 20°C und der Siedetemperatur des Lösungsmittels bei Normaldruck durchführt.

18. Verfahren nach Anspruch 11 zur Herstellung von 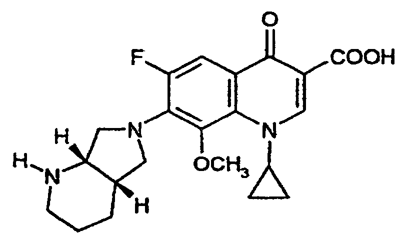 **dadurch gekennzeichnet, daß** man 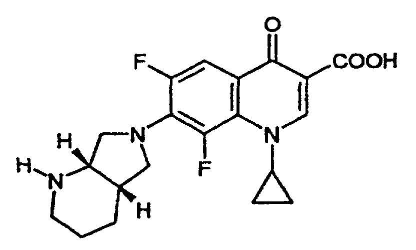 mit Methanol und Kalium-tert.-butylat in Tetrahydrofuran als Lösungsmittel umsetzt.

19. Verfahren nach Anspruch 18, **dadurch gekennzeichnet, daß** man bezogen auf ein Äquivalent der Verbindung der Formel 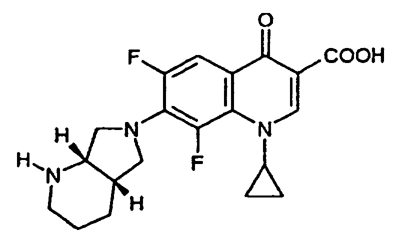 1 bis 3 Äquivalente Methanol und 2 bis 3 Äquivalente Kalium-tert.-butylat einsetzt.

20. Verfahren nach irgendeinem der Anspruch 18 oder 19, **dadurch gekennzeichnet, daß** man die Reaktion zwischen 20°C und der Siedetemperatur des Lösungsmittels bei Normaldruck durchführt.

21. Verfahren zur Herstellung von 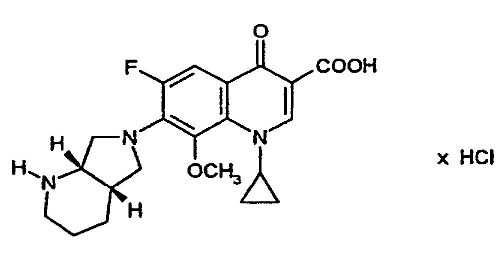 nach irgendeinem der Ansprüche 18 bis 21, **dadurch gekennzeichnet, daß** man nach der Umsetzung die Reaktionsmischung mit verdünnter Salzsäure versetzt oder die Reaktionsmischung zu verdünnter Salzsäure gibt und das ausgefallene Hydrochlorid durch Filtration isoliert und gegebenenfalls das isolierte Hydrochlorid durch Umkristallisation aus Wasser oder einem Wasser/C₁-C₃-Alkanol-Gemisch reinigt.

22. Verfahren nach Anspruch 21, worin aus Wasser oder einem Wasser/Ethanol-Gemisch umkristallisiert wird.

23. Verfahren zur Herstellung von 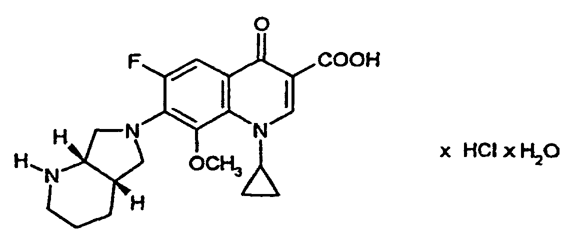 worin das gemäß Anspruch 22 erhaltene Produkt bei 40 bis 60°C und 80 bis 120 mbar getrocknet wird.

24. Verfahren nach Anspruch 23, worin die Trocknung bei etwa 50°C und etwa 100 mbar durchgeführt wird.

## Claims

1. Process for preparing 3-quinolonecarboxylic acid derivatives of the general formula 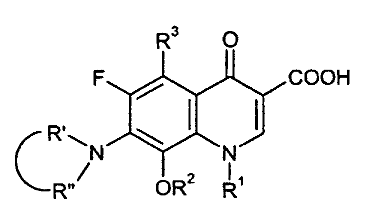 in which
R' and R" together with the linking nitrogen atom form a mono- or bicyclic heterocycle which may optionally contain in all ring moieties further nitrogen, oxygen or sulphur heteroatoms and which may optionally be substituted,
in which
R¹ represents C₁-C₃-alkyl, FCH₂-CH₂-, cyclopropyl, or represents phenyl or cyclopropyl, each of which is optionally mono- to trisubstituted by halogen,
R² represents C₁-C₃-alkyl or benzyl,
R³ represents H, halogen, NH₂, CH₃,
**characterized in that** 8-halogeno-3-quinolonecarboxylic acid derivatives of the general formula 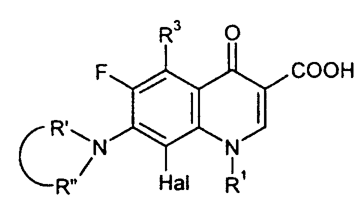 in which
Hal represents fluorine or chlorine and R¹, R³ and 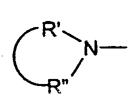
are each as defined above,
are reacted in an aliphatic or cycloaliphatic ether having 4 to 6 carbon atoms as solvent in the presence of C₁-C₃-alkanols or benzyl alcohol with 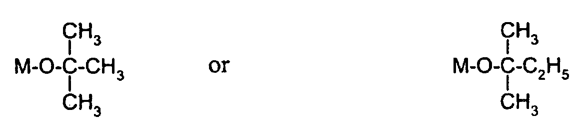 in which
M represents sodium or potassium.

2. Process according to Claim 1, **characterized in that** the solvent is selected from the group consisting of dimethoxyethane, dioxane and tetrahydrofuran.

3. Process according to Claim 2, **characterized in that** the solvent is tetrahydrofuran.

4. Process according to any of Claims 1 to 3, **characterized in that** Hal represents fluorine.

5. Process according to any of Claims 1 to 4, **characterized in that** the (C₁-C₃)-alkanol is methanol.

6. Process according to any of Claims 1 to 5, **characterized in that** M is potassium.

7. Process according to any of Claims 1 to 6, **characterized in that** based on 1 equivalent of the compound of the formula 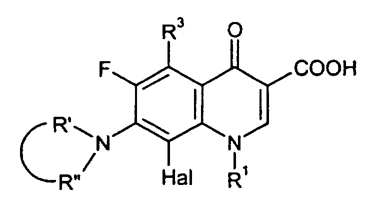 1 to 3, equivalents of the (C₁-C₃)-alkanol or the benzyl alcohol, and 2 to 3, equivalents of the compound of the formula 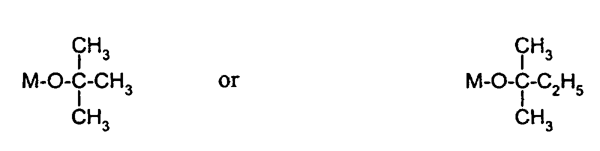 are employed.

8. Process according to any of Claims 1 to 7, **characterized in that** the reaction is carried out between 20°C and the boiling point of the solvent at atmospheric pressure.

9. Process for preparing 3-quinolonecarboxylic acid derivatives according to any of Claims 1 to 8 in which 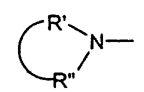 represents

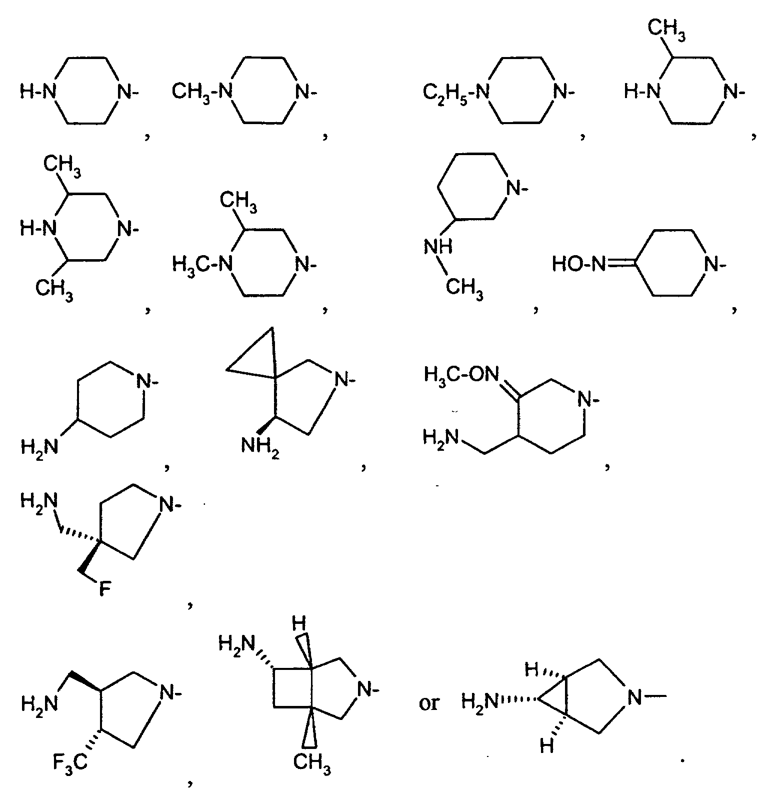

10. Process for preparing 3-quinolonecarboxylic acid derivatives according to any of Claims 1 to 8 in which 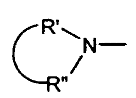 represents 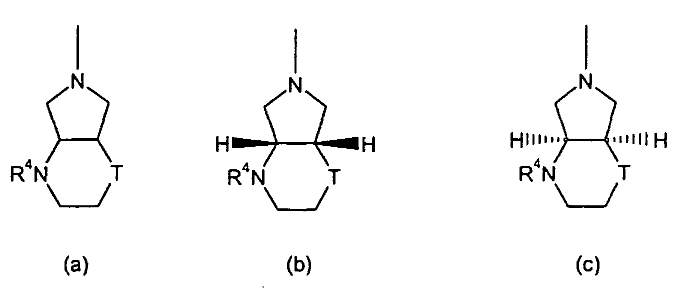 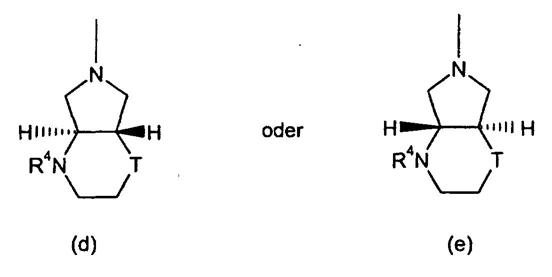 in which
T represents -O- or -CH₂- and
R⁴ represents hydrogen, C₁-C₃-alkyl, C₂-C₅-oxoalkyl, -CH₂-CO-C₆H₅, -CH₂CH₂CO₂R⁵, 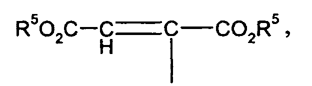 5-methyl-2-oxo-1,3-dioxol-4-yl-methyl, -CH=CH-CO₂R⁵ or -CH₂CH₂-CN,
in which
R⁵ represents hydrogen or C₁-C₃-alkyl,
and the formula (a) includes any mixtures of the stereoisomers (b) to (e).

11. Process for preparing 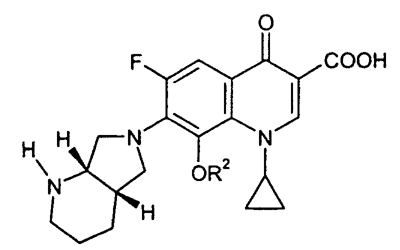 in which
R² represents C₁-C₃-alkyl or benzyl,
**characterized in that** 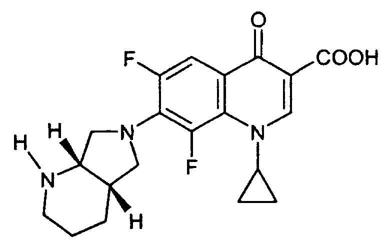 is reacted in an aliphatic or cycloaliphatic ether having 4 to 6 carbon atoms as solvent in the presence of C₁-C₃-alcohols or benzyl alcohol with 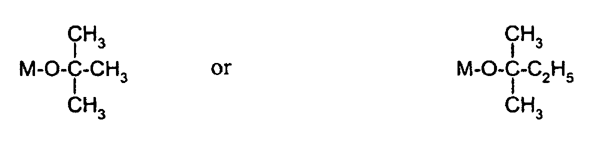 in which
M represents sodium or potassium.

12. Process according to Claim 11, **characterized in that** the solvent is selected from the group consisting of dimethoxyethane, dioxane and tetrahydrofuran.

13. Process according to Claim 11, **characterized in that** the solvent is tetrahydrofuran.

14. Process according to Claim 11, **characterized in that** the (C₁-C₃)-alkanol is methanol.

15. Process according to Claim 11, **characterized in that** M is potassium.

16. Process according to Claim 11, **characterized in that** based on 1 equivalent of the compound of the formula 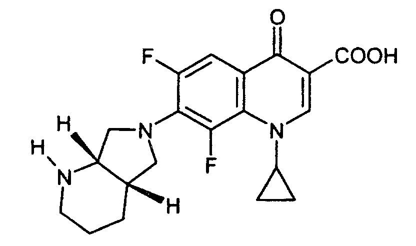 1 to 3 equivalents of the (C₁-C₃)-alkanol or the benzyl alcohol and 2 to 3, equivalents of the compound of the formula 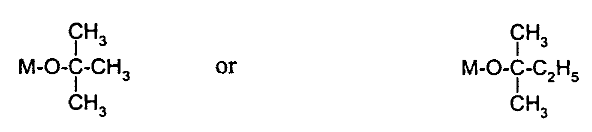 are employed.

17. Process according to Claim 11, **characterized in that** the reaction is carried out between 20°C and the boiling point of the solvent at atmospheric pressure.

18. Process according to Claim 11 for preparing 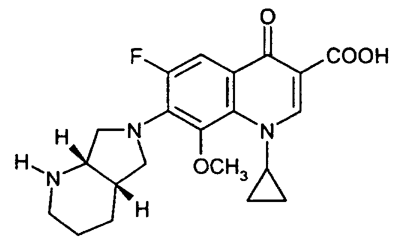 **characterized in that** 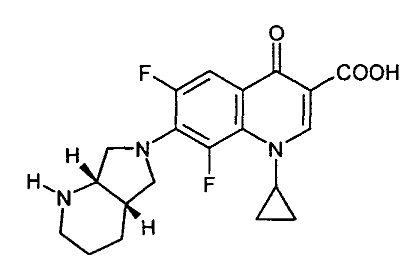 is reacted with methanol and potassium tert-butoxide in tetrahydrofuran as solvent.

19. Process according to Claim 18, **characterized in that** based on one equivalent of the compound of the formula 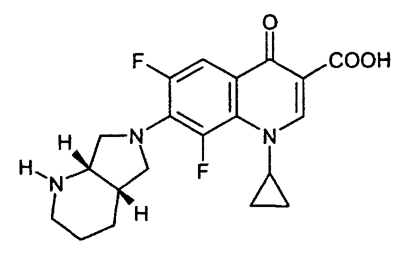 1 to 3 equivalents of methanol and 2 to 3, equivalents of potassium tert-butoxide are employed.

20. Process according to any of Claims 18 or 19, **characterized in that** the reaction is carried out between 20°C and the boiling point of the solvent at atmospheric pressure.

21. Process for preparing 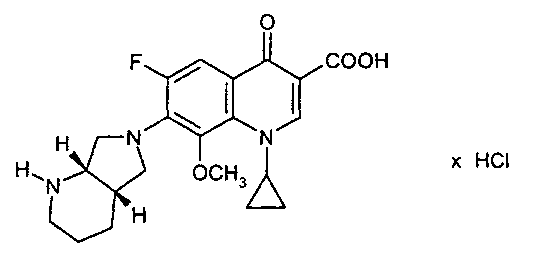 according to any of Claims 18 to 20, **characterized in that** after the reaction the reaction mixture is admixed with dilute hydrochloric acid or the reaction mixture is added to dilute hydrochloric acid and the precipitated hydrochloride is isolated by filtration and the isolated hydrochloride is if, appropriate, purified
by recrystillization from water or a water/C₁-C₃-alkanol mixture.

22. Process according to Claim 21, where the recrystillization is carried out from water or a water/ethanol mixture.

23. Process for preparing 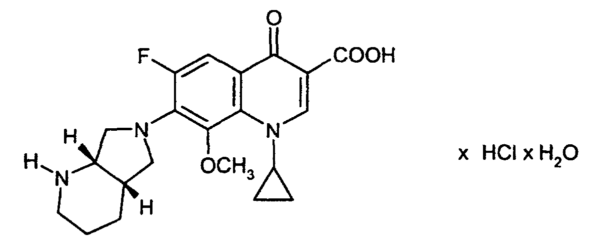 where the product obtained according to Claim 22 is dried at from 40 to 60°C and from 80 to 120 mbar.

24. Process according to Claim 23, where drying is carried out at approximately 50°C and approximately 100 mbar.

## Revendications

1. Procédé de production de dérivés d'acide 3-chinolonecarboxylique de formule générale 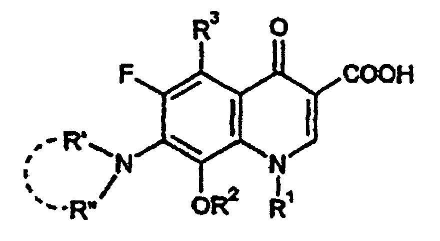 dans laquelle
R' et R" forment, conjointement avec l'atome d'azote auquel ils sont liés, un hétérocycle monocyclique ou bicyclique qui peut éventuellement contenir d'autres hétéroatomes d'azote, d'oxygène ou de soufre dans toutes les parties cycliques et qui peut éventuellement être substitué ,
dans laquelle
R¹ est un reste alkyle en C₁ à C₃, un groupe FCH₂-CH₂-, un reste phényle ou cyclopropyle éventuellement substitué une à trois fois par un halogène,
R² est un reste alkyle en C₁ à C₃ ou benzyle,
R³ représente H, un halogène, un groupe NH₂, CH₃,
**caractérisé en ce qu'**on fait réagir des dérivés d'acide 8-halogéno-3-chinolonecarboxylique de formule générale 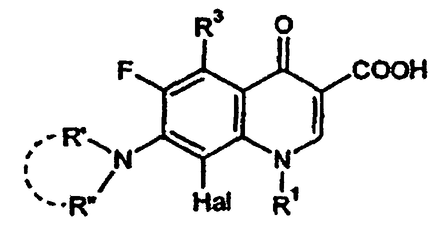 dans laquelle
Hal représente le fluor ou le chlore et R¹, R³ et 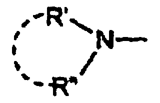 ont la définition indiquée ci-dessus,
dans un éther aliphatique ou cycloaliphatique ayant 4 à 6 atomes de carbone utilisé comme solvant en présence d'alcanols en C₁ à C₃ ou d'alcool benzylique, avec 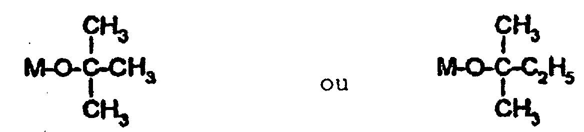 où
M représente le sodium ou le potassium.

2. Procédé suivant la revendication 1, **caractérisé en ce que** le solvant est choisi dans le groupe qui est constitué du diméthoxyéthane, du dioxanne et du tétrahydrofuranne.

3. Procédé suivant la revendication 2, **caractérisé en ce que** le solvant est le tétrahydrofuranne.

4. Procédé suivant l'une quelconque des revendications 1 à 3, **caractérisé en ce que** Hal représente le fluor.

5. Procédé suivant l'une quelconque des revendications 1 à 4, **caractérisé en ce que** l'alcanol en C₁ à C₃ est le méthanol.

6. Procédé suivant l'une quelconque des revendications 1 à 5, **caractérisé en ce que** M est le potassium.

7. Procédé suivant l'une quelconque des revendications 1 à 6, **caractérisé en ce qu'**on utilise, pour 1 équivalent du composé de formule 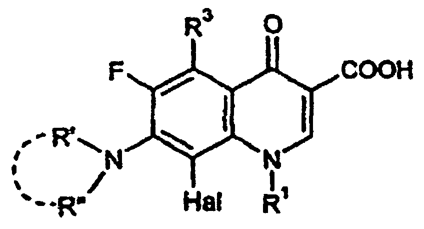 1 à 3 équivalents de l'alcanol en C₁ à C₃ ou de l'alcool benzylique et 2 à 3 équivalents du composé de formule

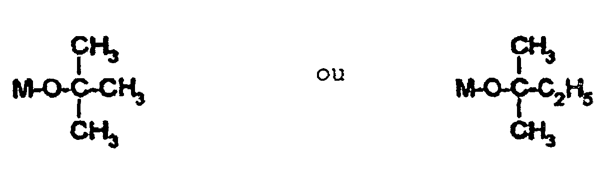

8. Procédé suivant l'une quelconque des revendications 1 à 7, **caractérisé en ce qu'**on conduit la réaction entre 20°C et le point d'ébullition du solvant, à la pression normale.

9. Procédé de production de dérivés d'acide 3-chinolonecarboxylique suivant l'une quelconque des revendications 1 à 8, où 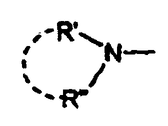 représente

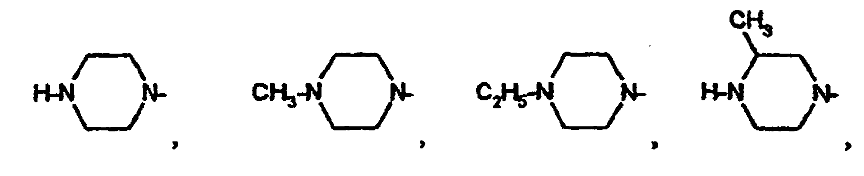

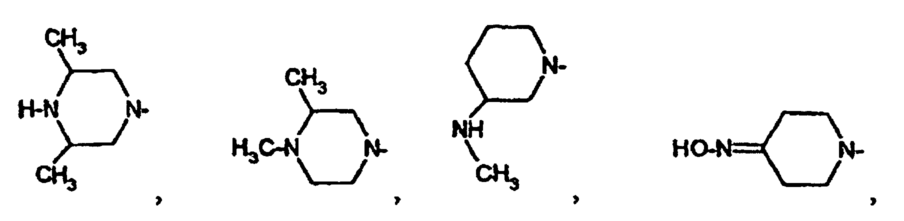

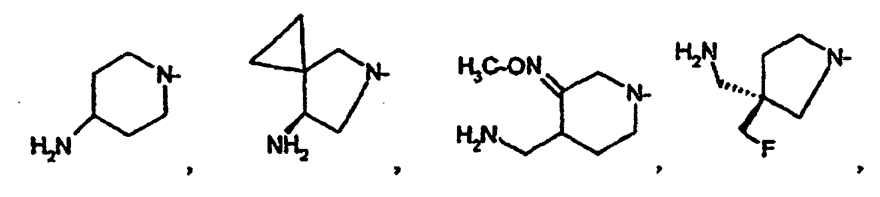

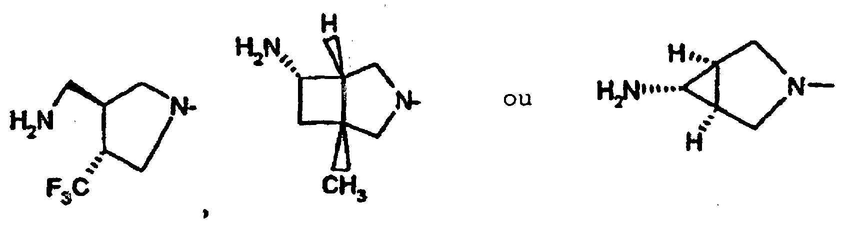

10. Procédé de production de dérivés d'acide 3-chinolonecarboxylique suivant l'une quelconque des revendications 1 à 8, où 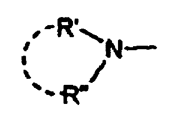 représente des groupes 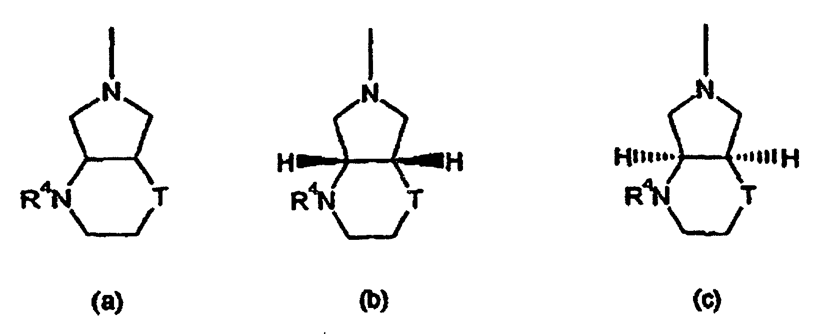 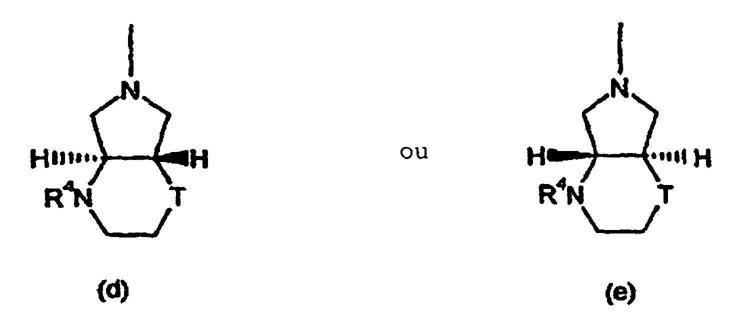 dans lesquels
T représente -O- ou -CH₂- et
R⁴ représente l'hydrogène, un reste alkyle en C₁ à C₃, oxoalkyle en C₂ à C₅, -CH₂-CO-C₆H₅, -CH₂CH₂CO₂R⁵, 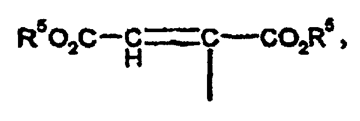 5-méthyl-2-oxo-1,3-dioxol-4-yl-méthyl, -CH=CH-CO₂R⁵ ou -CH₂CH₂-CN,
où
R⁵ représente l'hydrogène ou un groupe alkyle en C₁ à C₃,
et la formule (a) comprend des mélanges quelconques des stéréoisomères (b) à (e).

11. Procédé de production de 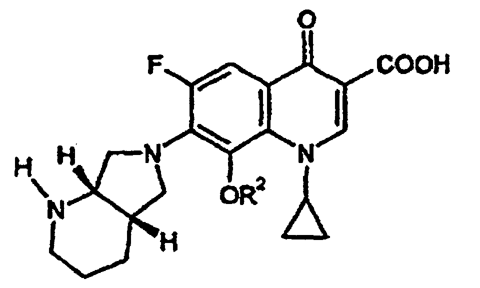 où
R² est un reste alkyle en C₁ à C₃ ou un reste benzyle,
**caractérisé en ce qu'**on fait réagir 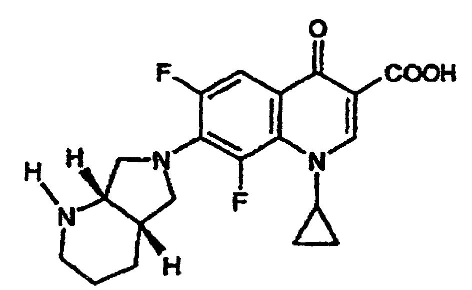 dans un éther aliphatique ou cycloaliphatique ayant 4 à 6 atomes de carbone utilisé comme solvant en présence d'alcools en C₁ à C₃ ou d'alcool benzylique, avec 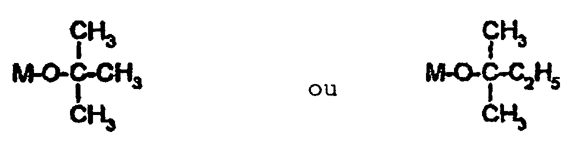 où
M représente le sodium ou le potassium.

12. Procédé suivant la revendication 1, **caractérisé en ce que** le solvant est choisi dans le groupe qui comprend le diméthoxyéthane, le dioxanne et le tétrahydrofuranne.

13. Procédé suivant la revendication 11, **caractérisé en ce que** le solvant est le tétrahydrofuranne.

14. Procédé suivant la revendication 11, **caractérisé en ce que** l'alcanol en C₁ à C₃ est le méthanol.

15. Procédé suivant la revendication 11, **caractérisé en ce que** M est le potassium.

16. Procédé suivant la revendication 11, **caractérisé en ce qu'**on utilise par équivalent de composé de formule 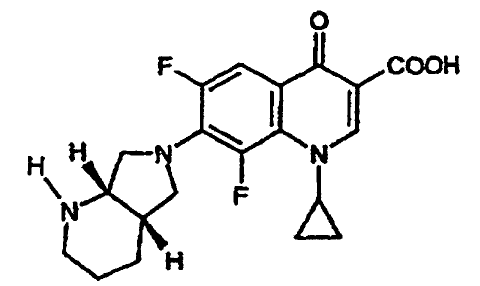 1 à 3 équivalents d'alcanol en C₁ à C₃ ou d'alcool benzylique et 2 à 3 équivalents du composé de formule

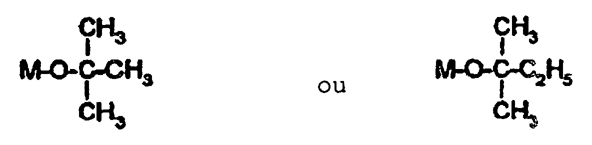

17. Procédé suivant la revendication 11, **caractérisé en ce qu'**on conduit la réaction entre 20°C et la température d'ébullition du solvant à la pression normale.

18. Procédé suivant la revendication 11, pour la production de 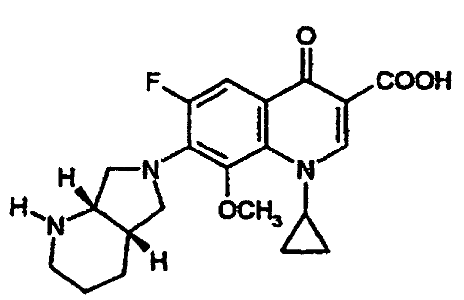 **caractérisé en ce qu'**on fait réagir 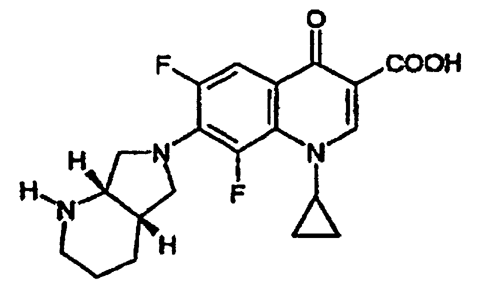 avec du méthanol et du tertiobutylate de potassium dans du tétrahydrofuranne utilisé comme solvant.

19. Procédé suivant la revendication 18, **caractérisé en ce qu'**on utilise, pour un équivalent de composé de formule 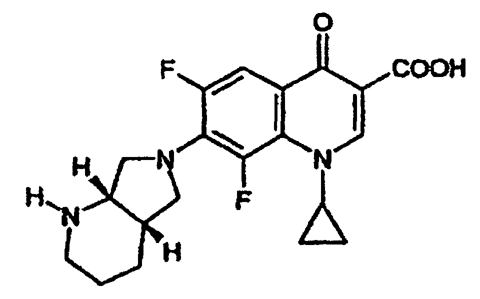 1 à 3 équivalents de méthanol et 2 à 3 équivalents de tertiobutylate de potassium.

20. Procédé suivant l'une quelconque des revendications 18 ou 19, **caractérisé en ce qu'**on conduit la réaction entre 20°C et la température d'ébullition du solvant à la pression normale.

21. Procédé de production de 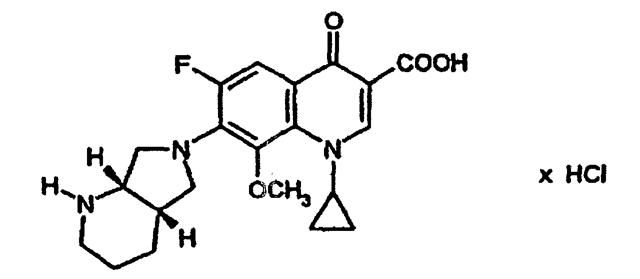 selon l'une quelconque des revendications 18 à 21, **caractérisé en ce qu'**on ajoute de l'acide chlorhydrique dilué au mélange réactionnel après la réaction ou bien on ajoute le mélange réactionnel à de l'acide chlorhydrique dilué et on isole par filtration le chlorhydrate précipité et on purifie éventuellement le chlorhydrate isolé par recristallisation dans l'eau ou dans un mélange d'eau et d'alcanol à C₁ à C₃.

22. Procédé suivant la revendication 21, dans lequel on conduit le recristallisation dans l'eau ou dans un mélange d'eau et d'éthanol.

23. Procédé de production de 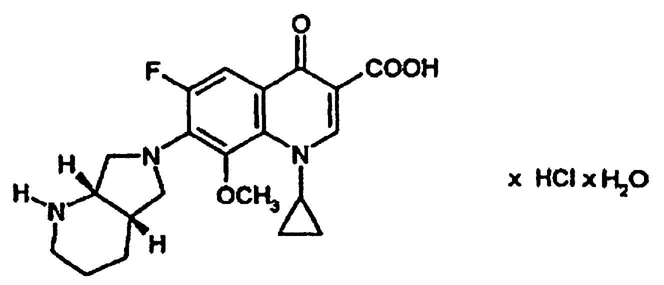 dans lequel le produit obtenu selon la revendication 22 est séché à une température de 40 à 60°C et une pression de 80 à 120 mbar.

24. Procédé suivant la revendication 23, dans lequel le séchage est conduit à environ 50°C et à environ 100 mbar.
